Europäisches Patentamt

European Patent Office

Office européen des brevets

(11) Publication number: 0 375 333 A2

# EUROPEAN PATENT APPLICATION

(21) Application number: 89313235.7

(51) Int. Cl.⁵: C07C 209/26

(22) Date of filing: 18.12.89

(30) Priority: 19.12.88 US 286419
19.12.88 US 286420

(43) Date of publication of application:
27.06.90 Bulletin 90/26

(84) Designated Contracting States:
AT BE CH DE ES FR GB GR IT LI LU NL SE

(71) Applicant: THE DOW CHEMICAL COMPANY
2030 Dow Center Abbott Road
Midland, MI 48640(US)

(72) Inventor: Tanis, Maarten
Ir v.Diggelenstraat 6
Hulst(NL)
Inventor: Rauniyar, Govind
Av. Clermont Tonnere 34
B-1330 Rixensart(BE)

(74) Representative: Burford, Anthony Frederick et al
W.H. Beck, Greener & Co. 7 Stone Buildings
Lincoln's Inn
London WC2A 3SZ(GB)

(54) Method for the reductive methylation of primary amines.

(57) Permethylated amines particularly pentamethyldiethylenetriamine, are prepared by a two stage reductive methylation of amines containing at least one primary amine group. In the first stage, only a sufficient amount of formaldehyde to monomethylate the primary amine groups present is continuously fed to the reaction system at a high flow rate, and thereafter the flow rate of the formaldehyde is substantially reduced for the second stage in which methylation is completed The production of by-products is substantially reduced and preparation times are significantly shorter in comparison to processes where the formaldehyde flow rate remains constantly high for the entirety of the reaction.

EP 0 375 333 A2

# METHOD FOR THE REDUCTIVE METHYLATION OF PRIMARY AMINES

This invention relates to a method for the reductive methylation of amines containing at least one primary amine group and has particular application in the production of permethylated amines.

In general, methylated amines are prepared by the reductive methylation of a suitable amine, for example, diethylenetriamine, using a reductive methylation catalyst, formaldehyde, and hydrogen.

Hitherto, such reactions have been carried out at relatively high pressures, for example, from 5000 to 20,000 KPa (50 to 200 bar). For example, DE 25 45 695-A and DE 26 18 580-A (BASF) both disclose reductive methylation processes, carried out at a pressure of 20,000 KPa (200 bar).

At such relatively high pressures, acceptably high yields can be obtained (for example from 90 to 95 percent). Commercially however, it is disadvantageous to utilize such high pressures, because of the need for relatively complex equipment and handling operations.

Japanese published Patent Application Kokai 60-130551 discloses a low pressure process for the reductive methylation of amines to produce tertiary amines. However, the process requires an expensive platinum or palladium catalyst.

It is therefore desirable to provide an economical method for the preparation of permethylated amines in good yield and purity avoiding the use of complex high pressure equipment and expensive metal catalysts.

We have discovered that reductive methylation of primary amines can be carried out in relatively high yield and economically convenient reaction times at low pressures, for example at 5,000 KPa (50 bar) or less, or even at pressures of 2,000 KPa (20 bar) or less, without the need for expensive catalysts or high pressure equipment.

In one aspect, this invention is a method for the preparation of N-methylated amines by superatmospheric pressure reductive methylation of amines initially containing at least one primary amine group which comprises reacting the amine with formaldehyde and hydrogen in the presence of a reductive methylation catalyst, wherein formaldehyde is fed continuously to the reaction mixture and characterized in that the flow rate of formaldehyde to the reaction system is reduced after a stoichiometric amount of formaldehyde sufficient to mono-methylate the primary amine groups in the first phase of the reaction has been fed but substantially before formaldehyde to methylate the so produced secondary amine groups is fed and wherein the flow rate to the reaction system is substantially reduced for the second phase during which a further quantity of formaldehyde sufficient to achieve complete methylation of the amine is fed.

Surprisingly, it has been found that conducting the reaction in such a way that the flow rate of the formaldehyde is reduced after the primary amine groups have been converted into secondary amine groups results in the preparation of permethylated amines in good yields and purity. Formation of undesirable byproducts is greatly decreased, as compared with conventional methods of operation, in which a constant formaldehyde flow rate is utilized. Further, the reaction time is greatly decreased in comparison to processes where the flow rate of the formaldehyde is held constant through the entirety of the preparation.

As described hereinabove, this invention relates to a method for the preparation of permethylated amines, and more particularly alkyl polyamines, wherein the flow rate of formaldehyde to the reaction system is reduced after the first phase consisting of mono-methylating the primary amine groups.

The extent to which the formaldehyde flow rate is reduced after the primary amine groups have been mono-methylated and converted into secondary amine groups is sufficient to result in substantially reduced by-product content in the resulting permethylated amines as compared to the same process wherein the aldehyde is supplied at a constant rate for the entirety of the preparation.

Typically, the reaction is carried out in two reaction rate phases, a first phase during which the stoichiometric amount of formaldehyde required for the mono-methylation of the said primary amine group(s) is fed to the reaction mixture at a substantially constant flow rate, and a second phase during which a further quantity of formaldehyde, sufficient to achieve complete methylation of all remaining amine hydrogens, is fed to the reaction system, at a flow rate substantially lower than the said substantially constant flow rate of the mono-methylation phase. Advantageously, the flow rate of formaldehyde to the reaction system during the first phase is reduced by at least 50 percent, preferably from 50 to 97.5 percent, and more preferably from 83 to 90 percent, for the second phase of the reaction.

Reducing the flow rate by percentages lower than this does not offer the significant reduction in by-product formation. By-products formed include, for example, partly methylated amine, higher molecular weight products and lower molecular weight products. Reductions of flow rates greater than 97.5 percent can be practiced but lead to very long overall reaction times.

It is important that the reduction of the flow rate of formaldehyde takes place when a stoichiometric quantity sufficient to mono-methylate the amine has been fed but substantially before any formaldehyde is fed that will give a permethylated product.

The duration of the first reductive methylation phase will depend on the overall reactivity of the primary amine group(s) and the number of primary amine groups to be reacted. The duration of the second phase depends on the reactivity of the secondary amine groups resulting and/or present in the amine starting material, but is preferably from 2 to 40 times that of the first phase, more preferably from 6 to 12 times that of the first phase.

For economic reasons, the flow rate of the formaldehyde for the first phase of the reductive methylation reaction is advantageously at least 0.1, depending on the reactivity of the amine, preferably at least 0.25 and more preferably at least 0.5 mole of formaldehyde per mole of amine per hour. Although it is possible to prepare products using slower feed rates, overall conversion and processing time become too long to be of commercial interest.

By way of example, in the reductive methylation of diethylenetriamine to produce pentamethyldiethylenetriamine, an amount of formaldehyde sufficient to mono-methylate the primary amine groups (40 percent of the stoichiometric quantity of formaldehyde required to provide the permethylated ethylene diamine) may be fed to the reaction system over a time period sufficient to achieve mono-methylation of the primary groups in the first phase of the reaction. Advantageously, this time period is from 0.5 to 4 hours, preferably from 0.5 to 2 hours. The remaining quantity of formaldehyde sufficient to achieve complete methylation of the remaining secondary amine groups is then fed in the second phase of the reaction. Advantageously, this duration of the second phase feed is from 1 to 40 hours, preferably from 12 to 20 hours depending on amine reactivity, and in most instances will be at least twice the period and preferably at least six times the period of the first phase.

Although the feed time for the first reaction period can be longer, such is not so commercially advantageous.

The method of the invention may be utilized for the preparation of various N-methylated aliphatic, acyclic, aromatic or heterocyclic amines. No special restrictions are imposed on the amine compounds which can be used as raw material in the method according to the invention, provided that they have at least one primary amine group and that they will undergo reductive methylation. Optionally, the amine compound may contain one or more secondary amine groups. The amines for which the process is particularly suitable are polyamines having in general from 1 to 30 carbon atoms and may be optionally substituted, the existing substituents of the amine can be branched or linear, and can include, for example, methyl, ethyl, isopropyl, or phenyl, and if the case arises may carry chemically reactive substituents which do not detrimentally affect the reductive methylation process such as, for example, chlorine.

Some representative examples of suitable amines are methylamine, ethylamine, propylamine, cyclohexylamine, ethylenediamine, propylenediamine (all isomers), diethylenetriamine, triethylenetetramine, polyalkylene polyamines such as tetraethylenepentamine, pentaethylenehexamine, aminoalkylpiperazines such as aminoethylpiperazine, alkanolamines such as monoethanolamine and aminoethylethanolamine, aniline, toluidene diamine, naphthylamine and mixtures thereof. A preferred amine for use in the method of this invention is ethylenediamine.

The term formaldehyde as used herein is intended to include within its scope both formaldehyde itself, and substances capable of decomposing to provide formaldehyde under the reaction conditions employed, for example trioxane and para-formaldehyde. The formaldehyde may be aqueous formalin or a formaldehyde solution in methanol. The formaldehyde concentration may be from 30 to 60 percent. Preferably the formaldehyde is 35 to 37 percent aqueous formalin. The quantity of formaldehyde used is at least an equimolar quantity in relation to each reactive hydrogen of nitrogen centre(s) of the amine. Generally, a quantity within the range of from 1.0 to 1.2 times the equimolar quantity in relation to each reactive hydrogen carried by the amine is used. In the description of this present invention the term reactive hydrogen refers to hydrogens associated directly with the nitrogen atom of the primary or secondary amine and which will undergo reductive methylation.

The reaction is carried out in the presence of a reductive methylation catalyst, many of which are known. Such catalysts generally comprise Ni, Pd, Co, Pt, or Cu. In the process of this invention, the preferred catalysts are those comprising nickel, cobalt or copper, and most preferred for reasons of economics are nickel catalysts. They may be employed as fixed bed catalysts or used in powdered form, whichever is convenient for the reactor and equipment available. Preferred nickel catalysts are those which are activated by relatively low temperatures, for example 80°C to 120°C. Examples of suitable nickel-containing catalysts are the commercially available hydrogenation/hydrogenolysis catalysts identified as Ni 5132P and Ni 6458P and sold by Harshaw Chemie (Nederland). The product Ni 5132P contains 64 percent by weight nickel on a proprietary support and has a surface area of $193m^2 \ g^{-1}$ and the product Ni 6458P contains 57 percent nickel, with a surface area of 204$m^2$ $g^{-1}$.

In the method of this invention, the ratio of catalyst to amine employed will generally depend on the nature of the amine employed its molecular weight and the catalyst employed, but is preferably within the range of from 0.1 to 30 g, more preferably from 4.0 to 8.0 g of catalyst/mole of amine. A lower catalyst concentration tends to reduce the hydrogenation rate to unacceptable levels, and thus lower the conversion. Higher catalyst concentrations increase the rate of hydrogenation, but tend to result in an increase in temperature, which results in unwanted side-reactions being favored, and thus an increased production of by-products.

The reaction is generally carried out at a temperature not exceeding 150°C, typically from 80°C to 150°C, preferably from 80°C to 120°C, and more preferably from 80°C to 100°C. Operating at higher reaction temperatures results in undesirable reaction products and at lower temperatures the rate of reaction can be very slow. The reaction is typically conducted at a pressure of from 300 KPa to 5,000 KPa (3 to 50 bar) or less, preferably 3,000 KPa (30 bar) or less, and more preferably 2,000 KPa (20 bar) or less. If the reaction is conducted at higher pressures, the distinct advantage of greatly reduced by-product formation through specific control of the formaldehyde feed rate is no longer significant.

It is particularly preferred in the method of the invention that the reaction is carried out in the presence of a volatile organic solvent. The volatile organic solvent should be one in which all the reactants are soluble and one which can readily be removed from the mixture at the end of the reductive methylation reaction.

Suitable solvents are those having a boiling point of 120°C or less and include aliphatic alcohols. Especially preferred are the aliphatic primary alcohols which include, for example, methanol, ethanol, propan-1-ol or mixtures thereof. Such solvents are preferred, because of the relatively higher solubility of hydrogen in them, as compared to water.

The ratio of the volatile organic solvent to the amine is preferably from 0.75:1 to 1.5:1 by weight, more preferably from 1.0:1 to 1.4:1 by weight, most preferably 1.2:1. The use of a volatile solvent enables the solvent to be readily removed by volatilization, to enable the product to be recovered.

The permethylated amine can be recovered by any of the techniques commonly employed which involve evaporation of the reaction medium and then distillation of the residue, the product being recovered as the distillate. However, such techniques suffer from the disadvantage that thermally unstable oily residues may accumulate.

A preferred procedure for recovering the permethylated amine obtained by the method of this invention is to extract the amine product from an aqueous reaction medium using a hydrocarbon in the presence of an ionic salting-out agent. After completion of the reductive-methylation procedure of this invention, the resulting composition comprises the permethylated amine in an aqueous alcoholic medium and solid catalyst residues. The volume of the resulting composition can be reduced by evaporation of the reaction medium, solvent, subsequent to filtering to remove the solid catalyst residues, and prior to extraction procedures.

Typically such ionic salting-out agents exhibit a high degree of solubility in polar solvents such as water or mixtures of alcohols with water, for example methanol. The salting-out agent ideally should not be reactive with the amine product and not be detrimental to the extraction process such as, for example, causing emulsions to form. The agent can be a neutral salt or strong base such as, for example, sodium chloride, potassium chloride, sodium hydroxide, potassium hydroxide, and the like or mixtures thereof. Preferred salting-out agents are sodium chloride, sodium hydroxide, or mixtures thereof.

Sufficient quantities of the salting-out agent are employed to enable full extraction of the permethylated amine. The quantities required will depend on the solubility of the amine in the reaction solvent, typically water and/or alcohol present in the reaction mixture and its ability to partition between this solvent and the extracting agent. Advantageously, the quantity of salting agent required is from 1 to 40, preferably from 10 to 35, and more preferably from 15 to 30 percent by weight of the total reaction mixture to be extracted including the salting agent. A quantity of salting agent lower than this gives little or no benefit to the efficiency of the extracting process, a quantity of salt higher than this may lead to agitation or processing complications.

The solvent of extraction, or extracting agent, to be used is one in which the permethylated amine shows good solubility, has a low solubility within the reaction medium or solvent, typically water and/or alcohol, and has a low boiling point enabling it to be readily removed from the permethylated amine after completion of the extraction. Suitable extraction solvents are the $C_{5-10}$ hydrocarbons, particularly the $C_{5-10}$ alkanes such as, for example, pentane, hexane, heptane, octane, nonane, and their various isomers, or mixtures thereof. Preferred are the $C_{5-7}$ alkanes which include pentane, hexane, heptane, and their various isomers, or mixtures thereof. Especially preferred are n-pentane and n-hexane due to their ready availability and suitable physical properties.

The extraction procedure, or recovery of permethylated amine, can be operated either as a batch extraction process or as a continuous extraction process.

In a batch extraction process, the reaction product containing the permethylated amine is treated with the salting-out agent and mixed with the extracting agent. The quantity of extracting agent employed is advantageously up to 100, preferably less than 60 volume percent of the volume of the reaction product and salting-out agent. When the permethylated amine partitions readily into the extracting agent, then volume percentages of extracting agent substantially lower than 60 percent can be employed.

After combining and mixing or agitating the reaction product, salting-out agent, and extracting agent, the complete mixture is allowed to stand to equilibrate prior to filtering to remove any insoluble solids such as the catalyst used during the permethylation step. The resulting filtrate will normally consist of an upper phase and a lower phase. Depending on the relative densities of the components, the upper phase will normally be the extracting agent containing permethylated amine product and the lower phase will normally be an aqueous or aqueous alcohol phase containing some permethylated amine product.

The two phases are separated and the aqueous or aqueous alcohol phase containing some permethylated amine product advantageously treated in a similar fashion again with the extracting agent. The resulting two phases are again separated and the aqueous or aqueous alcohol phase again treated with extracting agent. This cycle is repeated continuously until little or no permethylated amine remains in the aqueous or aqueous alcohol phase. Typically, as many as five or six extraction cycles may be necessary to arrive at the end- point of the extraction. The endpoint of the extraction can be determined by any suitable analytical method, for example, using gas-liquid chromatographic analysis to observe the concentration of permethylated amine product in the aqueous or aqueous alcohol phase.

Advantageously, all fractions of the extracting agent containing therein recovered permethylated amine product are combined and the extracting agent removed. The extracting agent can be suitably removed by evaporation, preferably at reduced pressures and elevated temperature. Reduced pressures ranging from 20 KPa (0.2 bar) to slightly less than 100 KPa (1 bar), preferably from 30 KPa (0.3 bar) to 70 KPa (0.7 bar), and more preferably from 30 KPa to 50 KPa (0.3 bar to 0.5 bar) are generally sufficient for the removal of the extracting agent. The elevated temperatures required to remove the extracting agent promptly will depend upon the particularly extracting agent used in the method but will always be less than the boiling point of this extracting agent at atmospheric pressure.

On removal of the extracting agent, a clean residue is obtained consisting essentially of the permethylated amine product. This residue can further, if desired, be fractionally distilled to obtain a highly purified product.

In a continuous extraction process, the reaction product containing the permethylated amine is first filtered to remove reductive methylation catalyst, and then treated with the salting-out agent. Similar proportions of salting-out agent, as with the batch extraction process, are employed. Prior to treatment with the salting-out agent, optionally the reaction solvent, typically alcohol and/or water, may be removed from the mixture containing the reaction product by evaporation at reduced pressures and/or elevated temperatures. Suitable temperatures and pressures are those which allow for the removal of the reaction solvent but not the permethylated amine.

The mixture comprising reaction product and salting-out agent is then subjected to a continuous extraction process by allowing the extracting agent to pass continuously through the said mixture. The theory of continuous extraction is described in, for example, *Textbook of Practical Organic Chemistry* by A. I. Vogel, pp. 127-138, 4th edition (1978) published by Longman Group Limited.

During the course of the continuous extraction process, the extracting agent becomes highly enriched in the permethylated amine and the treated reaction mixture, i.e., the raffinate, is nearly or totally depleted of permethylated amine.

The permethylated amine is isolated from enriched extracting agent by removing the extracting agent as described for the batch extraction process. The extracting agent can be recovered and reused in either one of the extraction procedures.

The permethylated amines prepared in accordance with the method of the invention, after recovery and isolation may be obtained in a purity of from 90 to 99 percent.

The permethylated amine compounds prepared in accordance with the process of this invention are useful industrial chemicals which can be applied in a wide application range, as intermediates for agricultural chemicals and medicines, as foaming catalysts for polyurethanes and curing agents for epoxy resins, emulsifying agents, dispersing agents, corrosion prevention agents, auxiliary agents for textile dyeing and as washing aids for example in the preparation of synthesis gas.

A number of preferred embodiments of the invention are described in the following examples. Unless otherwise stated, all quantities are parts or percentage by weight.

## Example 1

A stainless steel autoclave is charged with 10 parts of water, 10 parts of a nickel catalyst sold by Harshaw Chemie under the tradename Ni 6458P, 200 parts of diethylenetriamine and 240 parts of methanol.

The autoclave is purged with nitrogen and hydrogen and then pressurized with hydrogen, to a pressure of about 1700 KPa (17 bar). The contents are then heated to 85° C and maintained at this temperature for a period of half an hour, at a pressure of about 1900 KPa (19 bar).

A 37 percent solution of formaldehyde in water, containing about 10 percent of methanol as a stabilizing agent, is then pumped into the reactor at a feed rate of 315 parts/hr for a period of about 1 hour.

The formaldehyde feed rate is then reduced by about 83 percent to a rate of 38.7 parts/hr, and a further 542 parts of the same formaldehyde solution is fed to the reaction system, over a period of about 14 hours, at a constant rate.

Samples are taken throughout the reaction and analyzed by gas chromatography, after sedimentation of catalyst, to monitor the progress of the reaction.

After a total of 858 parts of formaldehyde have, as observed by GPC, been fed to the autoclave, the process provides a yield of pentamethyldiethylenetriamine on the basis of total organics (excluding methanol and formaldehyde), of 89.8 percent.

## Example 2

An autoclave is charged with 200 parts of diethylenetriamine, 10 parts of the nickel-containing catalyst Ni 6458P and 300 parts of water. The autoclave is then purged with nitrogen and hydrogen and then pressurized with hydrogen to a pressure of 1700 KPa (17 bar). The contents are then heated to a temperature of 85° C and maintained at this temperature for a period of about 30 minutes.

A 37 percent solution of formaldehyde in water, containing about 10 percent of methanol as a stabilizing agent, is then pumped into the reactor at a flow rate of 118 parts/hr for a period of about 2.5 hours.

After 295 parts of formaldehyde solution (37 percent of the stoichiometric amount required to give the permethylated product) has been fed, the formaldehyde flow rate is reduced by about 52.5 percent to a rate of 56 parts/hr. A further 618 parts of the same formaldehyde solution are then fed to the reaction over a period of about 11 hours.

At the end of the reaction, analysis of the products (excluding water and formaldehyde) showed the following product distribution: 29.4 percent partly methylated diethylenetriamine, 20.6 percent high molecular weight products, 5.7 percent low molecular weight products and 44.3 percent pentamethyldiethylenetriamine.

Viewing this example in comparison to Example 1 and Comparative Example A emphasizes the importance, for yield of product, of sufficiently reducing the flow rate of formaldehyde for the second phase of the preparative method.

## Comparative Example A

This example demonstrates the greatly inferior yield and purity when formaldehyde is continued to be fed at a high flow rate after the mono-methylation of the primary amines has been effected.

A stainless steel autoclave is charged with 10 parts of water, 10 parts of a nickel catalyst sold by Harshaw Chemie under the tradename Ni 6458P, 200 parts of diethylenetriamine and 240 parts of methanol.

The autoclave is purged with nitrogen and hydrogen and then pressurized with hydrogen, to a pressure of about 1700 KPa (17 bar). The contents are then heated to 85° C and maintained at this temperature for a period of half an hour, at a pressure of about 1900 KPa (19 bar).

A 37 percent solution of formaldehyde in water, containing about 10 percent of methanol as a stabilizing agent, is then pumped into the reactor at a flow rate of 315 parts/hr for a period of about 1.5 hours.

After 481 parts of formaldehyde (60 percent of the stoichiometric amount required to give the permethylated product) has been fed, the formaldehyde flow rate is reduced by about 82 percent to 56 parts/hr and a further 445 parts of the same formaldehyde solution is fed to the reaction over a period of about 8 hours.

After a total of 926 parts of formaldehyde have been fed to the autoclave, the process provides a yield of pentamethyldiethylenetriamine on the basis of total organics (excluding methanol and formaldehyde), of 17.5 percent. In addition 29.9 percent partly methylated diethylenetriamine, 43.1 percent high molecular weight products and 9.5 percent low molecular weight products are produced.

Even though the flow rate of formaldehyde is reduced by about 82 percent for the second feeding phase, there are substantial levels of undesirable by-products present as the high feed rate of formaldehyde is continued after the stoichiometric quantity to mono-methylate the amine has been fed. This comparative example emphasizes the critical period when it is necessary to reduce the flow, feed rates of formaldehyde as taught by this invention.

Comparative Example B

This comparative example illustrates the lower yields and purity of permethylated product obtained when formaldehyde is fed at a slow and constant feed rate for the entirety of the preparation.

An autoclave is charged and prepared as in Example 2.

A 37 percent solution of formaldehyde in water, containing about 10 percent of methanol as a stabilizing agent is then pumped into the reactor at a flow rate of 36.5 parts/hr for a period of about 22.7 hours, until a total of about 829 parts formaldehyde has been fed.

At the end of the reaction, analysis of the products (excluding water and formaldehyde) shows the following product distribution: 1.5 percent partly methylated diethylenetriamine, 11.4 percent high molecular weight products, 4.6 percent low molecular weight products and 82.6 percent pentamethyldiethylenetriamine.

A reasonable yield of pentamethyldiethylene triamine is obtained but the overall preparation time is now considerably longer than that for this invention, see Example 1.

Example 3 - Recovery of the Permethylated Compound, PMDETA

The crude reaction product as obtained in Example 1 is filtered at 20°C and 40 KPa (0.4 bar) pressure to give 1277 parts of a product containing 46 weight percent water, 22 weight percent methanol and 25 weight percent pentamethyldiethylenetriamine (PMDETA). Methanol is then removed from the filtered reaction mixture by distillation at ambient pressures and temperatures from about 64°C to 90°C to give 946 parts of a methanol-free composition comprising 62 weight percent water and 33.7 weight percent PMDETA.

A batch extraction process is carried out on this product. To 946 parts of the methanol-free composition, is added 254 parts of sodium hydroxide (in the form of pellets) and the mixture stirred with 560 parts of hexane at ambient temperature. The mixture is filtered and the upper organic, hexane, layer separated from the lower aqueous phase. The organic layer is retained and the aqueous phase extracted a further three times with hexane. The first and second repeat extractions are with 558 parts hexane and the third repeat extraction with 400 parts hexane.

The retained hexane extracts containing the PMDETA therein are combined and the hexane removed by distillation at a temperature of 50°C to 60°C and a reduced pressure of 0.1 to 0.3 bar. On removal of the hexane, 298 parts of a residue is obtained, gas-liquid chromatographic analysis of this residue indicates that it is PMDETA with a purity of 95 percent. The overall yield of the PMDETA from the reductive methylation procedure followed by this extraction method is about 85 percent.

Example 4

The recovery procedure of Example 3 is repeated four times on other portions of the reaction mixture, except that various amounts, respectively, of sodium hydroxide are added to the methanol-free crude reaction mixture, in respective runs, before extraction with hexane. The quantities of sodium hydroxide employed and the results of the batch extractions are shown in Table I.

TABLE I

| Recovery of PMDETA from the reactor mixture, by extraction with hexane, after filtration of catalyst and removal of methanol | | | | | | | |
|---|---|---|---|---|---|---|---|
| | A (wt %) | | | | | | |
| Sample # | PMDETA | CH$_3$OH | B (wt %) | Extr. step (#) | C (%) | D (%) | E (wt %) |
| 1 | 32.1 | - | 9.2 | 1 | 44.14 | 95.2 | |
| | | | | 2 | 22.75 | | |
| | | | | 3 | 13.70 | | |
| | | | | 4 | 5.50 | | |
| | | | | total | 86.00 | | <6.1 |
| 2 | 32.1 | - | 15.6 | 1 | 77.81 | - | |
| | | | | 2 | 9.96 | | |
| | | | | 3 | 1.48 | | |
| | | | | 4 | - | | |
| | | | | total | 89.30 | | <4.5 |
| 3 | 32.1 | - | 21.1 | 1 | 78.97 | 91.8 | |
| | | | | 2 | 11.47 | | |
| | | | | 3 | 1.71 | | |
| | | | | 4 | - | | |
| | | | | total | 92.10 | | <2.7 |
| 4 | 32.1 | - | 29.9 | 1 | 90.50 | 91.8 | |
| | | | | 2 | 1.53 | | |
| | | | | 3 | - | | |
| | | | | total | 92.10 | | <2.7 |

A Feed composition of PMDETA and CH$_3$OH before NaOH addition - balance is water

B NaOH added calculated as 100 · NaOH/(NaOH + feed)

C PMDETA extracted based on PMDETA in reactor mixture - analysis by titration

D Purity of PMDETA calculated as 100 · PMDETA/(all DETA derivatives) (gas-liquid chromatography)

E PMDETA in final raffinate

From the data it can be seen that using greater quantities of sodium hydroxide in the batch extraction process improves the efficiency of the extraction with a higher percentage of PMDETA being extracted.

Example 5

A crude methylated amine is prepared by the reductive methylation process of Example 1 and then subjected to a batch extraction process. The procedure employed for Example 3 and 4 is followed except that methanol is not removed to give a methanol-free reaction product. The batch extraction process is conducted with various respective amounts of sodium hydroxide present. The results of these extractions are shown in Table II.

TABLE II

| Recovery of PMDETA from the reactor mixture, by extraction with hexane, after filtration of catalyst | | | | | | | |
|---|---|---|---|---|---|---|---|
| | A (wt %) | | | | | | |
| Sample # | PMDETA | CH$_3$OH | B (wt %) | Extr. step (#) | C (%) | D (%) | E (wt %) |
| 5 | 25.7 | 22.6 | 20.9 | 1 | 87.17 | 95.2 | |
| | | | | 2 | 3.84 | | |
| | | | | 3 | 0.40 | | |
| | | | | 4 | - | | |
| | | | | total | 91.50 | | 2.5 |
| 6 | 25.7 | 22.6 | 30.7 | 1 | 85.25 | 87.7 | |
| | | | | 2 | 10.86 | | |
| | | | | 3 | 1.43 | - | |
| | | | | 4 | - | | |
| | | | | total | 97.50 | | 1.0 |

A Feed composition of PMDETA and CH$_3$OH before NaOH addition - balance is water

B NaOH added calculated as 100·NaOH/(NaOH + feed)

C PMDETA extracted based on PMDETA in reactor mixture - analysis by titration

D Purity of PMDETA calculated as 100·PMDETA/(all DETA derivatives) (gas-liquid chromatography)

E PMDETA in final raffinate

The results again indicate a significant gain in the efficiency of the extraction process is to be obtained by using the higher quantities of sodium hydroxide.

Example 6

A crude reaction mixture containing permethylated amine product is obtained as in Example 1. To 101 parts of this crude filtered reaction mixture is added 14 parts of sodium hydroxide pellets and this resulting mixture introduced into a continuous extractor. The resulting mixture is extracted for 5 to 6 hours with 92 parts of hexane to remove the PMDETA. The extractant, hexane, is then distilled off at 50°C to 60°C and 10 to 30 KPa (0.1 to 0.3 bar), to leave the pure isolated PMDETA. The PMDETA isolated has a purity by gas-liquid chromatography of 92 percent and the overall yield of the reductive methylation process and extraction is about 85 percent.

Example 7

The procedure of Example 6 is followed using a continuous extraction process and different quantities of sodium hydroxide. The results are shown in Table III, Samples 7 and 10.

Similar experiments were run using a continuous extraction process only in this case methanol was removed to give a methanol-free crude reaction product. Results of the continuous extraction process using various respective quantities of sodium hydroxide, are shown in Table III, Samples 11 to 12.

TABLE III

| | A (wt %) | | | | | |
|---|---|---|---|---|---|---|
| | Recovery of PMDETA from the reactor mixture, by continuous extraction with hexane, after filtration of catalyst and, optionally, removal of methanol | | | | | |
| Sample # | PMDETA | CH$_3$OH | B (wt %) | C (%) | D (%) | E (wt %) |
| 7* | 26.3 | 22.0 | 0.0 | 78.4 | 93.9 | 4.0 |
| 8 | 26.3 | 22.0 | 1.1 | 82.4 | 95.2 | 1.3 |
| 9 | 26.3 | 22.0 | 3.6 | 83.7 | 94.6 | 0.5 |
| 10 | 26.3 | 22.0 | 12.1 | 85.5 | 91.5 | 0.1 |
| 11* | 44.3 | 1.8 | 0.0 | 77.9 | 92.9 | 4.1 |
| 12 | 44.3 | 1.8 | 4.7 | 83.5 | 90.6 | 0.5 |

* Not an example of this invention, no ionic salting-out agent
A Feed composition of PMDETA and CH$_3$OH before NaOH addition - balance is water
B NaOH added calculated as 100*NaOH/(NaOH + feed)
C PMDETA extracted based on PMDETA in reactor mixture - analysis by titration
D Purity of PMDETA calculated as 100*PMDETA/(all DETA derivatives) (gas-liquid chromatography)
E PMDETA in final raffinate

As can be seen from the data in Table III, the efficiency of the extraction process is again improved by having higher quantities of sodium hydroxide present.

In comparing the data for the batch extraction procedure and continuous extraction procedure, it can be seen that the overall yield and purity of PMDETA obtained is equivalent. However, the continuous extraction process offers the advantage of giving this purity and extraction efficiency with substantially lower requirement of the salting-out agent.

The method of this invention can be used for the extraction and purification of alkylated, permethylated amines without necessitating the distillation of crude reaction mixtures containing the alkylated amine products, at high temperatures, and, without necessitating the caustic treatment at high temperatures of crude reaction mixtures containing alkylated amines where risks of explosion may be encountered.

**Claims**

1. A method for the preparation of completely N-methylated amines by superatmospheric pressure reductive methylation of amines initially containing at least one primary amine group which comprises reacting the amine with formaldehyde and hydrogen in the presence of a reductive methylation catalyst in a reaction medium, wherein the formaldehyde is fed continuously to the reaction and characterized in that only an amount of formaldehyde sufficient to monomethylate the primary amine groups is fed to the reaction in a first stage and then the flow rate of formaldehyde is substantially reduced for a second stage during which a further quantity of formaldehyde sufficient to achieve complete methylation of the amine is fed.

2. A method as claimed in Claim 1, wherein the amine is an alkyl polyamine and optionally contains secondary amine groups.

3. A method as claimed in Claim 1, wherein the flow rate of formaldehyde for the first stage of the reaction is reduced by at least 50 percent for the second stage of the reaction.

4. A method as claimed in Claim 3, wherein the flow rate of formaldehyde for the first stage is reduced by 83 to 90 percent for the second stage of the reaction.

5. A method as claimed in any one of the preceding claims, wherein the duration of the second stage is from 2 to 40 times that of the first stage.

6. A method as claimed in any one of the preceding claims wherein the duration of the second stage is from 6 to 12 times that of the first stage.

7. A method as claimed in any one of the preceding claims wherein the reaction is carried out in the presence of a volatile organic solvent.

8. A method as claimed in Claim 7, wherein the volatile organic solvent is a primary alcohol.

9. A method as claimed in any one of the preceding claims wherein the amine is ethylene diamine.

10. A method as claimed in any one of the preceding claims, wherein the reaction is carried out at a temperature not exceeding 150°C and wherein the rection is conducted at a pressure not exceeding 5000 KPa (50 bar).

11. A method as claimed in any one of the preceding claims, wherein the completely methylated product is recovered by extracting the product from the reaction medium with a $C_{5-10}$ alkane in the presence of an ionic salting-out agent.